# EUROPEAN PATENT SPECIFICATION

(11) **EP 4 507 644 B1**
(45) Date of publication and mention of the grant of the patent: **16.09.2026**
(21) Application number: 23718359.5
(22) Date of filing: 04.04.2023
(51) Int. Cl.: A61F 13/00, A61F 13/02

(54) **COAGULATION MOLD AND MANUFACTURING PROCESS THEREOF**
KOAGULATIONSFORM UND HERSTELLUNGSVERFAHREN DAFÜR
MOULE DE COAGULATION ET PROCÉDÉ DE FABRICATION ASSOCIÉ

(30) Priority: 12.04.2022 IL 29218922
(43) Date of publication of application: 19.02.2025
(73) Proprietor: Reddress Ltd., 3701142 Pardes Hana (IL)
(72) Inventor: KUSHNIR, Alon, 3780800 Givat Ada (IL)
(74) Representative: Klöckner, Christoph
(86) International application number: PCT/IL2023/050357
(87) International publication number: WO 2023/199310

(56) References cited:
- US-A1- 2005 089 551
- US-A1- 2020 281 775

## Description

### TECHNOLOGICAL FIELD

The present disclosure is in the field of medical devices, in particular medical devices that makes use of blood.

### BACKGROUND ART

References considered to be relevant as background to the presently disclosed subject matter are listed below:
- US 9,180,142
- WO 2019/058375
- WO 2021/124317
- US 2020/281775 discloses a wound dressing assembly including (i) blood-clotting mold device having an enclosure defined between walls of a main body and a removable closure over an opening and configured for introduction of blood thereinto, and (ii) coagulation initiator in amount sufficient to coagulate blood introduced into the enclosure to form a blood clot.
- US 2005/089551 discloses a window dressing that has a self-contained, ready to use blood clotting agent.

Acknowledgement of the above references herein is not to be inferred as meaning that these are in any way relevant to the patentability of the presently disclosed subject matter.

### GENERAL DESCRIPTION

The present disclosure provides a blood coagulation mold assembly and a method for manufacturing it. The blood coagulation mold assembly is intended to be filled whole blood and to allow the coagulation of the blood within a mold cavity thereof to obtain a blood clot that later is being applied on an injured portion for assisting in the recovery of it. For example, the clot that is formed in the blood coagulation mold assembly may be applied on any type of skin lesion to enhance its recovery. The coagulation mold assembly comprises a coagulation mold having a mold cavity containing one or more coagulation initiators, in which a blood is introduced to form a blood clot, thereby constituting a coagulation space. The coagulation mold comprises a peripheral portion extending from walls of the mold cavity in an outward direction with respect to the mold cavity. A blood-clot supporting matrix is formed over an opening of the mold cavity to allow its contact with the blood in the cavity such that the formed blood clot within the cavity is adhered to the supporting matrix. A removable closure, suitable for maintaining sterile conditions within the mold cavity is attached to the coagulation mold in two attachment portions including an inner attachment portion and an outer attachment portion. The inner attachment portion encircles the mold cavity and parts of the supporting matrix are disposed in the inner attachment portion, namely the support matrix is sandwiched between the coagulation mold and the removable closure and is retained in the desired position. The inner attachment portion further provides a sealing for the blood that is introduced into the mold cavity so as to maintain it confined to the mold cavity. It is to be noted that this attachment does not require to provide hermetic seal, only to ensure that the vast majority of the blood remains within the mold cavity. However, in some embodiments, the inner attachment seals blood from being transferred from the mold cavity through the inner attachment to peripheral portions. The outer attachment portion encircles the inner attachment portion and provides a sterile seal so as to maintain the mold cavity sterile.

The invention is set out in the appended set of claims.

Therefore, a first aspect of the present disclosure is provided any one of the following two configurations.

The first configuration provides a coagulation mold assembly. The coagulation mold assembly comprising a coagulation mold that comprises a mold cavity defined between walls of a main body and has an opening that is defined by the end portions of the walls of the main body. The mold cavity is configured for allowing introduction of blood thereinto. The introduction of the blood can be made by injection of blood through the walls defining the mod cavity or via an intended port that is formed in the walls defining the mold cavity. The coagulation mold further comprises a peripheral portion extending from the entire periphery of the end portion of the walls of the main body and is integral with the main body and is generally planar. The peripheral portion extends outwardly from the mold cavity and forming a suitable placement surface for placing the coagulation mold assembly.

A removable closure is formed over the opening of the mold cavity and over at least a part of the peripheral portion. The removable closure is intended to be removed after blood is coagulated in the mold cavity, thereby exposing the blood clot and allowing to apply the blood clot on a desired target, e.g. a skin lesion.

The coagulation mold assembly is further comprising a blood-clot support matrix or sheet that is retained between the mold cavity and the removable closure such that at least a first part of it is positioned over the opening and at least a second part of it is positioned over the peripheral portion. The removable closure is attached to the peripheral portion of the coagulation mold in two attachment portions encircling the mold cavity including an outer attachment portion wherein the attachment is directly between the removable closure and the coagulation mold for obtaining sterile conditions within the mold cavity, and an inner attachment portion between the outer attachment portion and the main body, wherein the blood-clot support matrix is intermediating in at least part of the inner attachment portion for holding the blood-clot support matrix in a desired position over the opening while forming the clot in the mold cavity.

This structure allows to introduce blood into a sterile mold cavity, through the walls of the main body either by piercing it with a syringe or via an intended port, and to allow the formation of a blood clot therein. The blood clot is adhered to the blood-clot support matrix and can be carried together with it. Once the blood clot is formed, the removable closure is removed and the exposed blood clot can be easily lifted by lifting the blood-clot support matrix from its peripheral portions that remains out of the mold cavity, namely the portions that are held by the inner attachment portion and peripheral thereto. Once the blood clot is lifted, it can be placed on any desired target, such as a skin lesion.

The second configuration provides a coagulation mold assembly. The coagulation mold comprising a coagulation mold that comprises a mold cavity that is defined between walls of a main body and has an opening, the end portions of the walls of the main body define the opening. The mold cavity is configured for introduction of blood thereinto. The coagulation mold further comprises a peripheral portion extending from the entire periphery of end portions of the walls of the main body and is integral with the main body and is generally planar. The peripheral portion extends outwardly from the mold cavity and forming a suitable placement surface for placing the coagulation mold assembly.

A removable closure is formed over the opening and over at least a part of the peripheral portion and is attached to the coagulation mold in at least two attachment portion at the peripheral portion including an inner attachment portion and an outer attachment portion. Each of the attachment portion is forming a closed frame, namely each of the attachment portion confines an inner space or inner portion. The inner attachment portion is formed between the outer attachment portion and the main body, and the outer attachment portion provides a sterile separation between the environment and the mold cavity.

A blood-clot support matrix or sheet is retained between the mold cavity and the removable closure in the inner attachment portion such that at least a first part of it is positioned over the opening and at least a second part of it is positioned over the peripheral portion.

After blood is introduced into the mold cavity, either through piercing of the walls of the main body or through an intended port formed in the walls, it is coagulated in the mold cavity and at least a part of it is held by or attached to the blood-clot support matrix or sheet. Then, the removable closure is removed to expose the blood clot and to allow its application, together with the blood-clot support matrix or sheet, on a desired target, such as a skin lesion.

It is to be noted that any combination of the described embodiments with respect to any aspect or configuration of this present disclosure is applicable. In other words, any aspect of the present disclosure can be defined by any combination of the described embodiments.

In some embodiments of the coagulation mold assembly, the mold cavity comprises coagulation initiator for initiating coagulation of blood being introduced thereinto.

In some embodiments of the coagulation mold assembly, the coagulation initiator is in powder form.

In some embodiments of the coagulation mold assembly, the coagulation initiator comprises a coagulation agent, an anti-coagulation agent, or both.

In some embodiments of the coagulation mold assembly, the anticoagulation agent is selected from a group consisting of: ACD-A (Anticoagulant Citrate Dextrose, Solution A), EDTA (ethylenediaminetetraacetic Acid), EGTA (ethylene glycol tetraacetic acid), a citrate, Heparin and oxalate.

In some embodiments of the coagulation mold assembly, the coagulation agent or the anti-anticoagulating agent is selected from: Kaolin, Ca²⁺, Mg²⁺, negatively charged phospholipid (PL) and protamine sulfate.

In some embodiments of the coagulation mold assembly, the walls of the main body defining said mold cavity are designed to allow introduction of blood into the mold cavity therethrough. For example, the blood can be introduced to the mold cavity by injecting the blood through the walls with a syringe.

In some embodiments of the coagulation mold assembly, a port is formed in the walls of the main body for allowing introduction of blood therethrough into the mold cavity.

In some embodiments of the coagulation mold assembly, the peripheral portion is integral with the main body.

In some embodiments of the coagulation mold assembly, the peripheral portion is generally planar making it suitable for placement on a surface to perform the coagulation process.

In some embodiments of the coagulation mold assembly, the blood-clot support matrix is a sheet or a gauze.

In some embodiments of the coagulation mold assembly, at least a part of the blood-clot support matrix is held or retained over the entire opening of the mold cavity.

In some embodiments of the coagulation mold assembly, the inner attachment portion is defined peripheral to the walls of the main body, namely the inner attachment portion is adjacent to the walls of the main body and encircling it.

In some embodiments of the coagulation mold assembly, the peripheral portion comprises a first abutment peripherally extending from the walls of the main body, specifically from the end portions of the walls. The first abutment is extending to a certain extent, defining a first abutment width. The first abutment defines the inner attachment portion, namely the attachment is made to the top portion of the first abutment.

In some embodiments of the coagulation mold assembly, the peripheral portion comprises a second abutment defining the outer attachment portion, namely the attachment is made to the top portion of the first abutment.

In some embodiments of the coagulation mold assembly, the removable closure is a synthetic flashspun high-density polyethylene fibers sheet.

In some embodiments of the coagulation mold assembly, the removable closure is a nonwoven product consisting of spun bond olefin fiber.

In some embodiments of the coagulation mold assembly, said attachment portions are formed by heat welding.

In some embodiments of the coagulation mold assembly, said attachment portions are formed by ultrasonic welding.

In some embodiments of the coagulation mold assembly, at least one of the walls of the mold cavity and closure is pierceable by needle or comprises a port, e.g. a unidirectional port, for allowing introduction of blood into the cavity.

In some embodiments of the coagulation mold assembly, the outer attachment portion seals contaminants from entering between the removable closure and the peripheral portion towards the mold cavity. Contaminants are considered as particles or organisms above a certain size that may adversely affect the blood clot that is formed in the mold cavity. The sealing that is formed by the outer attachment portion ensures that the mold cavity remains sterile until blood is introduced thereinto.

In some embodiments of the coagulation mold assembly, the outer attachment portion provides a sterile separation between the environment and the mold cavity. The coagulation mold and the removable closure are made of materials that are suitable for providing a sterile partition, and the outer attachment provides attachment between the peripheral portion and the removable closure in a sterile manner, ensuring that the mold cavity remains sterile until blood is introduced thereinto.

Yet another aspect of the present disclosure provides a method for manufacturing a coagulation mold assembly. The method comprising:
(i) providing a coagulation mold that comprises a mold cavity defined between walls of a main body and has an opening that is defined by the end portions of the walls of the main body. The mold cavity is configured for introduction of blood thereinto. The coagulation mold further comprises a peripheral portion extending from the entire periphery of end portions of the walls of the main body in an outward direction to the mold cavity. The peripheral portion is integral with the main body and is generally planar.
(ii) Placing a blood-clot support matrix on the coagulation mold such that at least a first part of it is placed over the opening and at least a second part of it is placed over the peripheral portion.
(iii) Attaching a removable closure to the peripheral portion in two attachment portions forming a closed frame encircling the mold cavity including an outer attachment portion wherein the attachment is directly between the removable closure and the coagulation mold for obtaining sterile conditions within the mold cavity, and an inner attachment formed portion between the outer attachment portion and the main body. The blood-clot support matrix is sandwiched between the peripheral portion and the removable closure in at least part of the inner attachment portion for holding the blood-clot support matrix in a desired position over the opening while forming the clot in the mold cavity.

In some embodiments, the method comprising introducing coagulation initiator into the mold cavity prior to said attaching.

In some embodiments of the method, the coagulation initiator is in powder form.

In some embodiments of the method, the coagulation initiator comprises a coagulation agent, an anti-coagulation agent, or both.

In some embodiments of the method, the anticoagulation agent is selected from a group consisting of: ACD-A (Anticoagulant Citrate Dextrose, Solution A), EDTA (ethylenediaminetetraacetic Acid), EGTA (ethylene glycol tetraacetic acid), a citrate, Heparin and oxalate.

In some embodiments of the method, the coagulation agent or the anti-anticoagulating agent is selected from: Kaolin, Ca²⁺, Mg²⁺, negatively charged phospholipid (PL) and protamine sulfate.

In some embodiments of the method, the walls of the main body defining said mold cavity are designed to allow introduction of blood into the mold cavity therethrough. For example, the blood can be introduced to the mold cavity by injecting the blood through the walls with a syringe.

In some embodiments of the method, a port is formed in the walls of the main body for allowing introduction of blood therethrough into the mold cavity.

In some embodiments of the method, the peripheral portion is integral with the main body.

In some embodiments of the method, the peripheral portion is generally planar.

In some embodiments of the method, the blood-clot support matrix is a sheet or a gauze.

In some embodiments of the method, said placing is performed such that at least a part of the blood-clot support matrix is placed over the entire opening of the mold cavity.

In some embodiments of the method, the inner attachment portion is defined peripheral to the walls of the main body, namely the inner attachment portion is adjacent to the walls of the main body and encircling it.

In some embodiments, the method comprising pressing the coagulation mold to the removable closure thereby forming a first abutment peripherally extending from the walls of the main body, the inner attachment portion is defined at said first abutment.

In some embodiments, the method comprising pressing the coagulation mold to the removable closure thereby forming a second abutment, the outer attachment portion is defined at said second abutment.

In some embodiments of the method, the removable closure is a flashspun high-density polyethylene fibers sheet.

In some embodiments of the method, the removable closure is a nonwoven product consisting of spun bond olefin fiber.

In some embodiments of the method, said attaching comprises heat welding said attachment portions.

In some embodiments of the method, said attaching comprises ultrasonic welding said attachment portions.

In some embodiments of the method, at least one of the walls of the mold cavity and closure is pierceable by needle or comprises a port, e.g. a unidirectional port, for allowing introduction of blood into the cavity.

In some embodiments of the method, said attaching results in a sealing that seals contaminants from entering between the removable closure and the peripheral portion towards the mold cavity. Contaminants are considered as particles or organisms above a certain size that may adversely affect the blood clot that is formed in the mold cavity. The sealing that is formed by the outer attachment portion ensures that the mold cavity remains sterile until blood is introduced thereinto.

In some embodiments of the method, said attaching results in a sterile separation between the environment and the mold cavity. The coagulation mold and the removable closure are made of materials that are suitable for providing a sterile partition, and the outer attachment provides attachment between the peripheral portion and the removable closure in a sterile manner, ensuring that the mold cavity remains sterile until blood is introduced thereinto.

### BRIEF DESCRIPTION OF THE DRAWINGS

In order to better understand the subject matter that is disclosed herein and to exemplify how it may be carried out in practice, embodiments will now be described, by way of non-limiting example only, with reference to the accompanying drawings, in which:

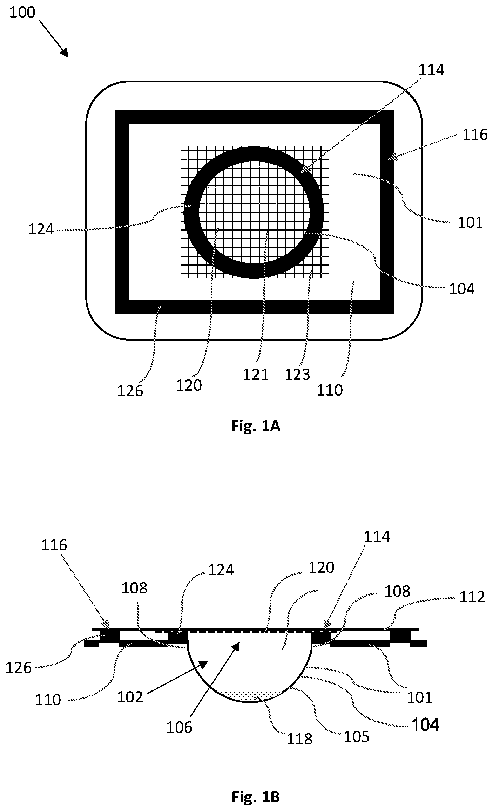

**Figs. 1A-1B** are schematic illustrations of different views of a non-limiting example of an embodiment of the coagulation mold assembly according to the present disclosure. **Fig. 1A** is a top view of the coagulation mold assembly and **Fig. 1B** is a cross sectional view of the coagulation mold assembly.

### DETAILED DESCRIPTION

The following figures are provided to exemplify embodiments and realization of the invention of the present disclosure.

Reference is being made to **Figs. 1A-1B****,** which are schematic illustrations of different views of a non-limiting example of an embodiment of the coagulation mold assembly according to the present disclosure. The coagulation mold assembly **100** comprising a coagulation mold **101** that comprises a mold cavity **102** defined between walls **104** of a main body **105,** and in this example, the walls **104** are concave. It is to be noted that the walls can be in any shape that confines a space. An opening **106** of the mold cavity **102** is defined by the end **108** of the walls **104.** The coagulation mold **101** further comprises a peripheral portion **110** extending from the end **108** of the walls **104** in a direction away from the mold cavity **102,** namely the peripheral portion **110** laterally extending away from the mold cavity **102.** The peripheral portion **110** is generally planar, except the attachment portions as detailed below.

A removable closure **112,** e.g. a flashspun high-density polyethylene fibers sheet film, is attached to the peripheral portion **110** in two attachment portions, an inner attachment portion **114** and an outer attachment portion **116.** The two attachment portions form a closed path, namely the attachment portions fully encircle the mold cavity **102,** each in a different distance therefrom. The removable closure **112** requires providing sterile separation between the environment and the internal of the mold cavity **102** prior to the use of the coagulation mold assembly **100.**

One or more coagulation initiators **118** are contained within the confined space of the mold cavity **102** so as to allow coagulation of blood when it is introduced thereinto. The blood can be introduced either by piercing a portion of the walls **104** of the mold cavity **102** or via a port, e.g. a unidirectional port formed in the walls **104.**

A blood-clot supporting matrix **120** is disposed between the removable closure **112** and the coagulation mold **101** such that at least a first part **121** of it is positioned over the opening **106** and a second part **123** of it is positioned at a portion of the inner attachment portion **114.** In this example, the blood-clot supporting matrix **120** is positioned along the entire circumference of the inner attachment portion **114.** Therefore, the attachment between the removable closure **112** and the coagulation mold **101** retains the blood-clot supporting matrix **120** in one or more portions. This configuration facilitates to maintain the blood-clot supporting matrix **120** in a suitable position such that it is submerged within or at least in contact with blood coagulating in the mold cavity **102** when blood is introduced to form a clot. Since the coagulation process is performed when the coagulation mold assembly **100** is placed on a surface such that the removable closure is facing the surface and engages it, the blood is found at the side of the blood-clot supporting matrix **120** and the formed clot is adhered to the blood-clot supporting matrix **120** to facilitate transformation of the blood clot onto the desired therapeutical target.

In this example, each of the two attachment portions **114** and **116** is constituted by abutments **124** and **126,** respectively, which are formed by heat press welding techniques. It is to be noted, that other attachment techniques can be used, such as ultrasonic welding, gluing, etc. The inner attachment portion **114** is circular and is formed at the circumference of the end **108** of the walls **104** of the mold cavity **102.** The outer attachment portion **116** is rectangular but it is to be understood that it can be in any closed shape that results in a sealed sterile space between the removable closure **112** and the coagulation mold **101** in any portion that is inwardly to the outer attachment portion **116** in the direction of the mold cavity **102,** namely all the portions between the outer attachment portion **116** and the mold cavity **102,** including, that some of them are sandwiched between the removable closure **112** and the peripheral portion **110.**

The purpose of the outer attachment portions **116** is to maintain the mold cavity sterile until it is used and the purpose of the inner attachment portion **114** is to retain the blood-clot supporting matrix **120** and prevent blood from leaking out of the mold cavity **102** during the coagulation process.

In order to use the coagulation mold assembly, whole blood is introduced into the mold cavity **102** and the coagulation process initiates. After the blood is coagulated and the blood-clot supporting matrix **120** is adhered thereto, the removable closure **112** is removed and the blood clot carried on the blood-clot supporting matrix **120** is transferred to be placed on the therapeutical target, e.g. a skin lesion.

## Claims

1. A blood coagulation mold assembly, comprising:
a coagulation mold that comprises
a mold cavity defined between walls of a main body and has an opening, the mold cavity is configured for introduction of blood thereinto, and
a peripheral portion extending from the entire periphery of an end of the walls of the main body;
a removable closure formed over the opening and at least a part of the peripheral portion and is attached to the coagulation mold in at least two attachment portion at the peripheral portion comprising an inner attachment portion and an outer attachment portion, each forming a closed frame, wherein the inner attachment portion is formed between the outer attachment portion and the main body and provides a sealing for the blood that is introduced into the mold cavity so as to maintain it confined to the mold cavity;
a blood-clot support matrix being retained between the mold cavity and the removable closure in the inner attachment portion such that at least a first part of it is positioned over the opening and at least a second part of it is positioned over the peripheral portion;
wherein the outer attachment portion provides a sterile separation between the environment and the mold cavity.

2. The coagulation mold assembly of claim 1, wherein the mold cavity comprises coagulation initiator for initiating coagulation of blood being introduced thereinto.

3. The coagulation mold assembly of claim 1 or 2, wherein the peripheral portion is integral with the main body; and
wherein the peripheral portion is generally planar.

4. The coagulation mold assembly of any one of claims 1-3, wherein the blood-clot support matrix is a sheet or a gauze; wherein at least a part of the blood-clot support matrix is held over the entire opening of the mold cavity.

5. The coagulation mold assembly of any one of claims 1-4, wherein the inner attachment portion is defined peripheral to the walls of the main body.

6. The coagulation mold assembly of any one of claims 1-5, wherein the peripheral portion comprises a first abutment peripherally extending from the walls of the main body, the first abutment defines the inner attachment portion.

7. The coagulation mold assembly of any one of claims 1-6, wherein the peripheral portion comprises a second abutment defining the outer attachment portion.

8. The coagulation mold assembly of any one of claims 1-7, wherein said attachment portions are formed by heat welding or ultrasonic welding.

9. The coagulation mold assembly of any one of claims 1-8, wherein at least one of the walls of the mold cavity and closure is pierceable by needle or comprises a port for allowing introduction of blood into the cavity.

10. The coagulation mold assembly of any one of claims 1-9, wherein the outer attachment portion seals contaminants from entering between the removable closure and the peripheral portion towards the mold cavity.

11. A method for manufacturing a blood coagulation mold assembly, comprising:
providing a coagulation mold that comprises
a mold cavity defined between walls of a main body and has an opening, the mold cavity is configured for introduction of blood thereinto, and
a peripheral portion extending from the entire periphery of an end of the walls of the main body;
placing a blood-clot support matrix on the coagulation mold such that at least a first part of it is placed over the opening and at least a second part of it is placed over the peripheral portion;
attaching a removable closure to the peripheral portion in two attachment portions encircling the mold cavity comprising an outer attachment portion and an inner attachment portion, wherein the inner attachment portion is formed between the outer attachment portion and the main body and provides a sealing for the blood that is introduced into the mold cavity so as to maintain it confined to the mold cavity, and wherein the blood-clot support matrix is sandwiched between the peripheral portion and the removable closure in at least part of the inner attachment portion, thereby being retained in position;
wherein said attaching results in a sterile separation between the environment and the mold cavity.

12. The method of claim 11, comprising introducing coagulation initiator into the mold cavity prior to said attaching.

13. The method of claim 11 or 12, further comprising:
pressing the coagulation mold to the removable closure thereby forming a first abutment peripherally extending from the walls of the main body, the inner attachment portion is defined at said first abutment; and
pressing the coagulation mold to the removable closure thereby forming a second abutment, the outer attachment portion is defined at said second abutment.

14. The method of any one of claims 11-13, wherein said attaching comprises either heat welding or ultrasonic welding said attachment portions.

15. The method of any one of claims 11-14, wherein said attaching results in a sealing that seals contaminants from entering between the removable closure and the peripheral portion towards the mold cavity.

## Patentansprüche

1. Blutgerinnungsformanordnung, umfassend:
eine Gerinnungsform, die umfasst
einen Formhohlraum, der zwischen Wänden eines Hauptkörpers definiert ist und eine Öffnung aufweist, wobei der Formhohlraum so konfiguriert ist, dass Blut in ihn eingeleitet werden kann, und
einen Randabschnitt, der sich von dem gesamten Rand eines Endes der Wände des Hauptkörpers aus erstreckt;
einen abnehmbaren Verschluss, der über der Öffnung und mindestens einem Teil des Randabschnitts ausgebildet ist und an der Gerinnungsform in mindestens zwei Befestigungsabschnitten am Randabschnitt befestigt ist, die einen inneren Befestigungsabschnitt und einen äußeren Befestigungsabschnitt umfassen, die jeweils einen geschlossenen Rahmen bilden, wobei der innere Befestigungsabschnitt zwischen dem äußeren Befestigungsabschnitt und dem Hauptkörper ausgebildet ist und eine Abdichtung für das in den Formhohlraum eingeführte Blut bereitstellt, um es so auf den Formhohlraum beschränkt zu halten;
eine Blutgerinnselträgermatrix, die zwischen dem Formhohlraum und dem abnehmbaren Verschluss im inneren Befestigungsabschnitt derart gehalten wird, dass mindestens ein erster Teil davon über der Öffnung positioniert ist und mindestens ein zweiter Teil davon über dem Randabschnitt positioniert ist;
wobei der äußere Befestigungsabschnitt eine sterile Trennung zwischen der Umgebung und dem Formhohlraum bereitstellt.

2. Gerinnungsformanordnung nach Anspruch 1, wobei der Formhohlraum einen Gerinnungsinitiator zum Initiieren der Gerinnung von darin eingeleitetem Blut umfasst.

3. Gerinnungsformanordnung nach Anspruch 1 oder 2, wobei der Randabschnitt integral mit dem Hauptkörper ist; und
wobei der Randabschnitt generell eben ist.

4. Gerinnungsformanordnung nach einem der Ansprüche 1-3, wobei die Blutgerinnselträgermatrix ein Blatt oder eine Gaze ist; wobei mindestens ein Teil der Blutgerinnselträgermatrix über der gesamten Öffnung des Formhohlraums gehalten wird.

5. Gerinnungsformanordnung nach einem der Ansprüche 1-4, wobei der innere Befestigungsabschnitt peripher zu den Wänden des Hauptkörpers definiert ist.

6. Gerinnungsformanordnung nach einem der Ansprüche 1-5, wobei der Randabschnitt einen ersten Anschlag umfasst, der sich peripher von den Wänden des Hauptkörpers aus erstreckt, wobei der erste Anschlag den inneren Befestigungsabschnitt definiert.

7. Gerinnungsformanordnung nach einem der Ansprüche 1-6, wobei der Randabschnitt einen zweiten Anschlag umfasst, der den äußeren Befestigungsabschnitt definiert.

8. Gerinnungsformanordnung nach einem der Ansprüche 1-7, wobei die Befestigungsabschnitte durch Heißschweißen oder Ultraschallschweißen geformt sind.

9. Gerinnungsformanordnung nach einem der Ansprüche 1-8, wobei mindestens eine der Wände des Formhohlraums und des Verschlusses durch eine Nadel durchstechbar ist oder eine Öffnung zum Einleiten von Blut in den Hohlraum umfasst.

10. Gerinnungsformanordnung nach einem der Ansprüche 1-9, wobei der äußere Befestigungsabschnitt gegen das Eindringen von Verunreinigungen zwischen dem abnehmbaren Verschluss und dem Randabschnitt in Richtung des Formhohlraums abdichtet.

11. Verfahren zum Herstellen einer Gerinnungsformanordnung, umfassend:
Bereitstellen einer Gerinnungsform, die umfasst
einen Formhohlraum, der zwischen Wänden eines Hauptkörpers definiert ist und eine Öffnung aufweist, wobei der Formhohlraum so konfiguriert ist, dass Blut in ihn eingeleitet werden kann, und
einen Randabschnitt, der sich von dem gesamten Rand eines Endes der Wände des Hauptkörpers aus erstreckt;
Platzieren einer Blutgerinnselträgermatrix auf der Gerinnungsform derart, dass mindestens ein erster Teil davon über der Öffnung platziert wird und mindestens ein zweiter Teil davon über dem Randabschnitt platziert wird;
Befestigen eines abnehmbaren Verschlusses am Randabschnitt in zwei Befestigungsabschnitten, die den Formhohlraum umgeben, die einen äußeren Befestigungsabschnitt und einen inneren Befestigungsabschnitt umfassen, wobei der innere Befestigungsabschnitt zwischen dem äußeren Befestigungsabschnitt und dem Hauptkörper ausgebildet ist und eine Abdichtung für das in den Formhohlraum eingeleitete Blut bereitstellt, um es so auf den Formhohlraum beschränkt zu halten, und wobei die Blutgerinnselträgermatrix in mindestens einem Teil des inneren Befestigungsabschnitts zwischen dem Randabschnitt und dem abnehmbaren Verschluss eingeklemmt ist, wodurch sie in Position gehalten wird;
wobei das Befestigen zu einer sterilen Trennung zwischen der Umgebung und dem Formhohlraum führt.

12. Verfahren nach Anspruch 11, umfassend das Einleiten eines Gerinnungsinitiators in den Formhohlraum vor dem Befestigen.

13. Verfahren nach Anspruch 11 oder 12, weiter umfassend:
Pressen der Gerinnungsform an den abnehmbaren Verschluss, wodurch ein erster Anschlag, der sich peripher von den Wänden des Hauptkörpers aus erstreckt, gebildet wird, wobei der innere Befestigungsabschnitt an dem ersten Anschlag definiert wird; und
Pressen der Gerinnungsform an den abnehmbaren Verschluss, wodurch ein zweiter Anschlag gebildet wird, wobei der äußere Befestigungsabschnitt an dem zweiten Anschlag definiert wird.

14. Verfahren nach einem der Ansprüche 11-13, wobei das Befestigen entweder Heißschweißen oder Ultraschallschweißen der Befestigungsabschnitte umfasst.

15. Verfahren nach einem der Ansprüche 11-14, wobei das Befestigen zu einer Abdichtung führt, die gegen das Eindringen von Verunreinigungen zwischen dem abnehmbaren Verschluss und dem Randabschnitt in Richtung des Formhohlraums abdichtet.

## Revendications

1. Ensemble moule pour coagulation sanguine, comprenant :
un moule de coagulation qui comprend
une cavité de moule définie entre les parois d'un corps principal et comportant une ouverture, cette cavité de moule étant configurée pour permettre l'introduction de sang à l'intérieur, et
une portion périphérique s'étendant depuis la périphérie totale d'une extrémité des parois du corps principal ;
une fermeture amovible formée sur l'ouverture et au moins une partie de la portion périphérique et qui est fixée au moule de coagulation en au moins deux portions de fixation au niveau de la portion périphérique comprenant une portion de fixation intérieure et une portion de fixation extérieure, chacune formant un cadre fermé, dans lequel la portion de fixation intérieure est formée entre la portion de fixation extérieure et le corps principal et assure l'étanchéité du sang introduit dans la cavité du moule afin de le maintenir confiné dans cette cavité ;
une matrice de support de caillot sanguin étant maintenue entre la cavité du moule et la fermeture amovible dans la portion de fixation interne de telle sorte qu'au moins une première partie de celle-ci soit positionnée au-dessus de l'ouverture et au moins une deuxième partie au-dessus de la portion périphérique ;
dans lequel la portion de fixation extérieure assure une séparation stérile entre l'environnement et la cavité du moule.

2. Ensemble moule de coagulation selon la revendication 1, dans lequel la cavité du moule comprend un initiateur de coagulation pour initier la coagulation du sang qui y est introduit.

3. Ensemble moule de coagulation selon la revendication 1 ou 2, dans lequel la portion périphérique est intégrante avec le corps principal ; et
dans lequel la portion périphérique est généralement plane.

4. Ensemble moule de coagulation selon l'une quelconque des revendications 1 à 3, dans lequel la matrice de support du caillot sanguin est une feuille ou une gaze ; dans lequel au moins une partie de la matrice de support du caillot sanguin est maintenue sur toute l'ouverture de la cavité du moule.

5. Ensemble moule de coagulation selon l'une quelconque des revendications 1 à 4, dans lequel la portion de fixation intérieure est définie comme étant périphérique aux parois du corps principal.

6. Ensemble moule de coagulation selon l'une quelconque des revendications 1 à 5, dans lequel la portion périphérique comprend une première butée s'étendant de manière périphérique à partir des parois du corps principal, la première butée définissant la portion de fixation intérieure.

7. Ensemble moule de coagulation selon l'une quelconque des revendications 1 à 6, dans lequel la portion périphérique comprend une deuxième butée définissant la portion de fixation extérieure.

8. Ensemble moule de coagulation selon l'une quelconque des revendications 1 à 7, dans lequel lesdites portions de fixation sont formées par soudage thermique ou par soudage aux ultrasons.

9. Ensemble moule de coagulation selon l'une quelconque des revendications 1 à 8, dans lequel au moins une des parois de la cavité du moule et de la fermeture est perforable par une aiguille ou comprend un orifice permettant l'introduction de sang dans la cavité.

10. Ensemble moule de coagulation selon l'une quelconque des revendications 1 à 9, dans lequel la portion de fixation extérieure empêche les contaminants de pénétrer entre la fermeture amovible et la portion périphérique vers la cavité du moule.

11. Procédé de fabrication d'un ensemble de moule pour coagulation sanguine, comprenant :
la fourniture d'un moule de coagulation qui comprend
une cavité de moule définie entre les parois d'un corps principal et comportant une ouverture, cette cavité de moule étant configurée pour permettre l'introduction de sang à l'intérieur, et
une portion périphérique s'étendant depuis la périphérie totale d'une extrémité des parois du corps principal ;
le placement d'une matrice de support de caillot sanguin sur le moule de coagulation de telle sorte qu'au moins une première partie soit placée sur l'ouverture et au moins une deuxième partie sur la portion périphérique ;
la fixation d'une fermeture amovible à la portion périphérique en deux portions de fixation entourant la cavité du moule comprenant une portion de fixation extérieure et une portion de fixation intérieure, dans lequel la portion de fixation intérieure est formée entre la portion de fixation extérieure et le corps principal et assure l'étanchéité du sang introduit dans la cavité du moule afin de le maintenir confiné dans la cavité du moule, et dans lequel la matrice de support du caillot sanguin est prise en sandwich entre la portion périphérique et la fermeture amovible dans au moins une partie de la portion de fixation intérieure, étant ainsi maintenue en position ;
dans lequel ledit dispositif de fixation permet une séparation stérile entre l'environnement et la cavité du moule.

12. Procédé selon la revendication 11, comprenant l'introduction d'un initiateur de coagulation dans la cavité du moule avant ladite fixation.

13. Procédé selon la revendication 11 ou 12, le procédé comprenant en outre :
la compression du moule de coagulation contre la fermeture amovible, formant ainsi une première butée s'étendant de manière périphérique à partir des parois du corps principal, la portion de fixation interne est définie à ladite première butée ; et
la compression du moule de coagulation contre la fermeture amovible, formant ainsi une deuxième butée, la portion de fixation extérieure est définie à ladite deuxième butée.

14. Procédé selon l'une quelconque des revendications 11 à 13, dans lequel ladite fixation comprend soit le soudage à chaud, soit le soudage par ultrasons desdites parties de fixation.

15. Procédé selon l'une quelconque des revendications 11 à 14, dans lequel ladite fixation permet d'obtenir une étanchéité qui empêche les contaminants de pénétrer entre la fermeture amovible et la portion périphérique vers la cavité du moule.
